# EUROPÄISCHE PATENTSCHRIFT

(11) **EP 2 671 548 B1**
(45) Veröffentlichungstag und Bekanntmachung des Hinweises auf die Patenterteilung: **26.12.2018**
(21) Anmeldenummer: 13166582.0
(22) Anmeldetag: 06.05.2013
(51) Int. Cl.: A61F 13/08, A47G 25/90, D04B 1/26

(54) **Kompressionsstrumpf und Anziehhilfe**
Compression stocking and donning aid
Bas de contention et aide à l'enfilage

(30) Priorität: 06.06.2012 DE 102012209564
(43) Veröffentlichungstag der Anmeldung: 11.12.2013
(73) Patentinhaber: BSN -Jobst GmbH, 46446 Emmerich (DE)
(72) Erfinder: Krimmel, Gerhard, 72766 Reutlingen (DE)
(74) Vertreter: Kohler Schmid Möbus Patentanwälte

(56) Entgegenhaltungen:
- EP-A1- 0 620 998
- DE-A1- 2 716 306
- DE-A1-102005 012 338
- GB-A- 2 260 686
- US-A- 3 905 212
- US-A- 3 975 929
- US-A- 5 412 957
- US-A1- 2009 218 377

## Beschreibung

Kompressionsstrümpfe haben die Aufgabe, von außen Druck auf das Gewebe des umschlossenen Beines auszuüben, um das Venen- oder Lymphsystem des Beines zu entlasten. Den erforderlichen Druck kann der Kompressionsstrumpf nur dann ausüben, wenn er entsprechend eng am Bein anliegt. Durch diesen engen Sitz ist es mit einigem Kraftaufwand verbunden, den Kompressionsstrumpf anzulegen. Insbesondere ältere und in ihrer Bewegung eingeschränkte Personen haben daher häufig Schwierigkeiten, einen Kompressionsstrumpf anzulegen. Dies gilt umso mehr, wenn der Kompressionsstrumpf eine geschlossene Spitze aufweist, d. h. auch die Zehen des Patienten umschließt. Außerdem kann das Anlegen des Kompressionsstrumpfes erschwert sein, wenn die Haut beispielsweise nach dem Duschen feucht ist oder Ekzeme oder Wunden aufweist.

Es sind daher schon Gestelle vorgeschlagen worden, mit denen der Strumpf aufgeweitet und über das Bein gezogen werden kann. Das Aufziehen des Strumpfes auf diese Gestelle erfordert jedoch ebenfalls einige Zeit und einiges Geschick. Außerdem sind die Gestelle sperrig und insbesondere auf Reisen daher nur umständlich zu gebrauchen.

Für Kompressionsstrümpfe mit offener Spitze, d. h. Strümpfe, die die Zehen des Patienten nicht umschließen, sind außerdem Anziehhilfen in Form von Gleitsocken bekannt, die nach dem Überziehen den Reibungswiderstand zwischen Fuß und Kompressionsstrumpf verringern und dadurch das Anlegen des Strumpfes erleichtern. Sobald der Strumpf am Bein sitzt, kann die Anziehhilfe durch die offene Spitze im Bereich der Zehen aus dem Strumpf herausgezogen werden.

Der vorliegenden Erfindung liegt die Aufgabe zugrunde, eine Möglichkeit zu schaffen, einen Kompressionstrumpf mit geschlossener Spitze einfacher anlegen zu können, als dies bisher der Fall war.

Die DE 27 16 306 C2, die GB 2 260 686 A, die US 3,905,212 A und die US 3,975,929 A beschreiben einen nach Operationen anzulegenden Antithrombosestrumpf mit einer relativ großen Öffnung im Sohlenbereich, der ein Umschlagen des Strumpfes und Freilegen der Zehen zu Untersuchungszwecken erlaubt, ohne dazu den Strumpf ausziehen zu müssen.

Aus der DE 10 2005 012 338 A1 ist ein Strumpf mit einer großen Öffnung im Sohlenbereich bekannt, wobei die Öffnung einen Durchtritt der Fußsohle im Zehenballenbereich erlaubt, um ein Ausgleiten beim Gehen mit den Strümpfen zu vermeiden.

Die Aufgabe wird gelöst durch ein Set bestehend aus einem Kompressionsstrumpf mit einer geschlossenen Spitze, die an ihrer Unterseite eine Öffnung aufweist, wobei die Öffnung nach Anlegen des Kompressionsstrumpfes im Bereich der Grundgelenke der Zehen des Patienten zu liegen kommt, und einer Anziehhilfe für einen Kompressionsstrumpf, die aus einem Stoff mit glatter Oberfläche gefertigt und vor Anlegen des Kompressionsstrumpfes über den Fuß des Patienten streifbar ist und durch die Öffnung im Spitzenbereich des Kompressionsstrumpfes nach Anlegen des Kompressionsstrumpfes herausziehbar ist.

Der Kompressionsstrumpf umschließt nach dem Anlegen auch die Zehen des Patienten, sodass er auch dort Druck ausüben kann, was bei einigen Anwendungen medizinisch erforderlich ist. Darüber hinaus sind Kompressionsstrümpfe mit geschlossenen Spitzen auch wärmer als Strümpfe mit einem offenen Zehenbereich. Durch die Öffnung auf der Unterseite der Spitze muss zum erleichterten Anlegen dieses Strumpfes nicht notwendigerweise eine der bekannten gestellartigen Anziehhilfen verwendet werden. Der Strumpf kann vielmehr auch mit Hilfe einer Anziehhilfe aus einem glatten Stoff in ähnlich einfacher Weise wie ein Kompressionsstrumpf mit offener Spitze angelegt werden. Sobald der Strumpf am Bein sitzt, kann die Anziehhilfe durch die Öffnung auf der Unterseite der Spitze des Kompressionsstrumpfes herausgezogen werden.

Die Öffnung des Kompressionsstrumpfes kommt nach Anlegen des Kompressionsstrumpfes im Bereich der Grundgelenke der Zehen des Patienten zu liegen. An dieser Stelle behindert die Öffnung weder das Tragen von Schuhen noch das Gehen. Auch die Kompressionswirkung des Strumpfes wird durch die Öffnung an dieser Stelle nicht eingeschränkt. Auch vollständig geschlossene Kompressionsstrümpfe liegen im Bereich der Grundgelenke der Zehen nicht am Fuß an und haben somit an dieser Stelle ebenfalls keine Kompressionswirkung.

Die Öffnung kann prinzipiell jede beliebige Form aufweisen. Vorzugsweise ist sie jedoch rund, um sowohl das Greifen als auch das Herausziehen der Anziehhilfe möglichst einfach zu gestalten.

Dabei sollte die Öffnung vorzugsweise im angelegten Zustand des Kompressionsstrumpfes eine Weite aufweisen, die ein Eindringen von zwei Fingerspitzen erlaubt. Die Öffnung muss dann nicht erst durch die Finger aufgeweitet werden, um die Anziehhilfe herausziehen zu können.

Weitere Vorteile ergeben sich, wenn der Öffnungsrand mit einem elastischen textilen Flächengebilde verstärkt ist. Diese Verstärkung verhindert ein Ausreißen des Öffnungsrandes und ein Bilden von Laufmaschen im Kompressionsstrumpf.

Dabei kann das Flächengebilde vorzugsweise aus einem mit klebenden Substanzen beschichteten, elastischen textilen Material bestehen, das unter Druck und Hitzeeinwirkung aufbringbar ist. Ein solches Flächengebilde lässt sich beispielsweise mit einer Heizplatte, mit Hilfe von Ultraschall oder hochfrequenten elektromagnetischen Wellen auf dem Kompressionsstrumpf aufbringen. Dabei kann die Öffnung auch vorzugsweise nach Aufbringen des Flächengebildes herstellbar sein. Das Flächengebilde kann dazu beispielsweise kreisförmig aufgebracht und anschließend die Öffnung durch Ausschneiden oder Ausstanzen eines kreisförmigen Teils mit kleinerem Durchmesser aus dem mit dem Flächengebilde bedeckten Bereich des Kompressionsstrumpfes hergestellt werden. Auf diese Weise ist eine Fertigung des erfindungsgemäßen Kompressionsstrumpfes relativ rasch und mit hoher Qualität möglich.

Durch die glatte Oberfläche der Anziehhilfe lässt sich der Kompressionsstrumpf deutlich leichter überziehen als direkt auf der Haut des Patienten, da die Haut einen deutlich höheren Reibungswiderstand für den Strumpf bildet als die Anziehhilfe.

Dabei ist es von Vorteil, wenn die Anziehhilfe aus einem glatten, in Quer- und Längsrichtung unelastischen Gewebe gefertigt ist. Dies stellt sicher, dass sich die Anziehhilfe beim Anlegen des Kompressionsstrumpfes nicht verziehen kann.

Die Anziehhilfe kann sockenartig oder pantoffelartig geformt sein. Dabei ist die Pantoffelform vorzuziehen, da die Anziehhilfe dann leichter anzulegen ist und nicht auf unterschiedliche Fußgrößen angepasst werden muss. Dennoch kann durch die Pantoffelform sichergestellt werden, dass der Kompressionsstrumpf auch über die Ferse des Patienten leicht hinweg gezogen werden kann, wenn die Anziehhilfe entsprechend lang ausgebildet wird.

Material und Größe der Anziehhilfe sollten vorzugsweise so gewählt werden, dass sie leicht durch die Öffnung im Spitzenbereich des Kompressionsstrumpfes nach Anlegen des Strumpfes herausziehbar ist. Auch das Herausziehen der Anziehhilfe wird durch deren glatte Oberfläche erleichtert. Der Reibungswiderstand zwischen der Haut des Patienten und der Anziehhilfe sowie zwischen der Anziehhilfe und dem Kompressionsstrumpf ist nur gering.

Selbstverständlich kann das Set auch zwei Kompressionsstrümpfe mit einer Anziehhilfe enthalten. Da der Patient die Strümpfe nacheinander anlegt, ist eine Anziehhilfe ausreichend.

Nachfolgend wird ein bevorzugtes Ausführungsbeispiel eines erfindungsgemäßen Kompressionsstrumpfes sowie einer Anziehhilfe anhand der Zeichnung näher beschrieben.

Es zeigen:
- Fig. 1: eine Ansicht eines Beines mit übergezogenem Kompressionsstrumpf mit Blick auf die Sohle;
- Fig. 2: eine Draufsicht auf eine Anziehhilfe.

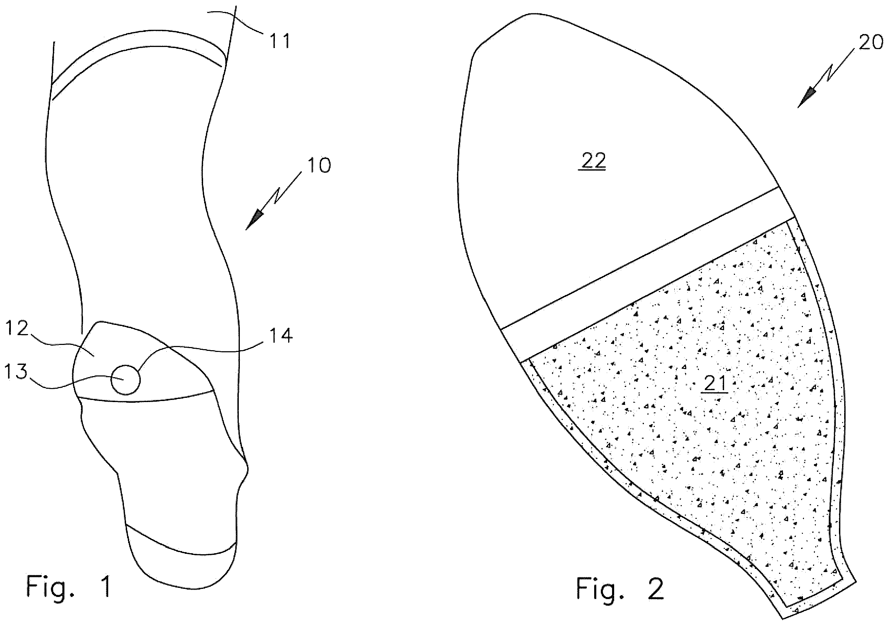

Der in Fig. 1 gezeigte Kompressionsstrumpf 10 umschließt das Bein 11 eines Patienten bis zum Oberschenkel. Der Strumpf 10 weist eine Spitze 12 auf, die bis auf eine Öffnung 13 geschlossen ist. Die Öffnung 13 befindet sich dabei auf der Unterseite der Spitze 12 im Bereich der Zehengrundgelenke. Sie weist eine runde Form auf, wobei der Öffnungsrand eine Verstärkung aufweist, die aus einem textilen Flächengebilde bestehen kann, das elastisch ist und das mit klebenden Substanzen beschichtet und unter Druck und Hitzeeinwirkung mit dem Material des Kompressionsstrumpfes 10 verbunden wurde. Zweckmäßigerweise kann das Flächengebilde kreisförmig aufgebracht und aus diesem kreisförmigen Bereich später die Öffnung 13 ausgeschnitten werden. Von dem Flächengebilde bleibt dann eine ringförmige Verstärkung 14 am Öffnungsrand übrig, die ein Ausreißen des Öffnungsrandes und Bilden von Laufmaschen verhindert.

Zur Erleichterung des Anlegens des Kompressionsstrumpfes 10 aus Fig. 1 kann eine Anziehhilfe 20 gemäß Fig. 2 verwendet werden. Die Anziehhilfe 20 ist pantoffelförmig ausgebildet mit einer taschenartigen Spitze 21 zur Aufnahme des Zehen- und Ristbereichs des Fußes des Patienten. Anschließend an die taschenförmige Spitze 21 erstreckt sich eine einflächige Verlängerung 22 des sohlenseitigen Teils dieser Spitze 21. Dieser Bereich 22 legt sich beim Überstreifen des Kompressionsstrumpfes 10 um die Ferse des Patienten, sodass auch dort aufgrund der glatten Oberfläche des Materials der Anziehhilfe 20 beim Anlegen des Kompressionsstrumpfes 10 nur ein geringer Reibungswiderstand herrscht. Ist der Kompressionsstrumpf 10 angelegt worden und sitzt er korrekt am Bein, so kann die Anziehhilfe 20 durch die Öffnung 13 des Kompressionsstrumpfes 10 herausgezogen werden. Auch das Herausziehen der Anziehhilfe 20 aus der Öffnung 13 wird durch deren glatte Oberfläche und das unelastische Material erleichtert.

## Patentansprüche

1. Set bestehend aus einem Kompressionsstrumpf mit einer geschlossenen Spitze (12), die an ihrer Unterseite eine Öffnung (13) aufweist, wobei die Öffnung (13) nach Anlegen des Kompressionsstrumpfes (10) im Bereich der Grundgelenke der Zehen des Patienten zu liegen kommt, und einer Anziehhilfe für einen Kompressionsstrumpf, die aus einem Stoff mit glatter Oberfläche gefertigt und vor Anlegen des Kompressionsstrumpfes (10) über den Fuß des Patienten streifbar ist und durch die Öffnung (13) im Spitzenbereich des Kompressionsstrumpfes (10) nach Anlegen des Kompressionsstrumpfes (10) herausziehbar ist.

2. Set nach Anspruch 1, **dadurch gekennzeichnet, dass** die Öffnung (13) eine runde Form aufweist.

3. Set nach Anspruch 1 oder 2, **dadurch gekennzeichnet, dass** die Öffnung (13) im angelegten Zustand des Kompressionsstrumpfes (10) eine Weite aufweist, die ein Eindringen von zwei Fingerspitzen erlaubt.

4. Set nach einem der Ansprüche 1 bis 3, **dadurch gekennzeichnet, dass** der Öffnungsrand (14) mit einem elastischen textilen Flächengebilde verstärkt ist.

5. Set nach Anspruch 4, **dadurch gekennzeichnet, dass** das Flächengebilde aus einem mit klebenden Substanzen beschichteten, elastischen textilen Material besteht, das unter Druck und Hitzeeinwirkung aufbringbar ist.

6. Set nach Anspruch 5, **dadurch gekennzeichnet, dass** das Flächengebilde mittels einer Heizplatte, mit Hilfe von Ultraschall oder hochfrequenten elektromagnetischen Wellen aufbringbar ist.

7. Set nach einem der Ansprüche 4 bis 6, **dadurch gekennzeichnet, dass** die Öffnung (13) nach Aufbringen des Flächengebildes herstellbar ist.

8. Set nach einem der Ansprüche 1 bis 7, **dadurch gekennzeichnet, dass** die Anziehhilfe aus einem glatten, in Quer- und Längsrichtung unelastischen textilen Stoff gefertigt ist.

9. Set nach einem der Ansprüche 1 bis 8, **dadurch gekennzeichnet, dass** die Anziehhilfe pantoffelartig geformt ist.

10. Set nach einem der Ansprüche 1 bis 9, **dadurch gekennzeichnet, dass** es einen zweiten Kompressionsstrumpf (10) enthält.

## Claims

1. Set consisting of a compression stocking having a closed toe (12) provided on its underside with an opening (13), wherein the opening (13) lies in the region of the metatarsophalangeal joints of the patient after the application of the compression stocking (10), and a compression stocking application aid made of a material having a smooth surface, wherein the application aid can be slipped over the foot of the patient before the application of the compression stocking (10) and removed through the opening (13) in the toe region of the compression stocking (10) after the application of the compression stocking (10).

2. Set according to claim 1, **characterised in that** the opening (13) has a round shape.

3. Set according to claim 1 or claim 2, **characterised in that**, when the compression stocking (10) is applied, the opening (13) has a width allowing for the passage of two fingertips.

4. Set according to one of claims 1 to 3, **characterised in that** the edge (14) of the opening is reinforced with an elastic textile fabric.

5. Set according to claim 4, **characterised in that** the fabric consists of an elastic textile material that is coated with adhesive substances and can be applied using pressure and heat.

6. Set according to claim 5, **characterised in that** the fabric can be applied by means of a hot plate, with the aid of ultrasound or high-frequency electromagnetic waves.

7. Set according to one of claims 4 to 6, **characterised in that** the opening (13) can be produced after the application of the fabric.

8. Set according to one of claims 1 to 7, **characterised in that** the application aid is made of a smooth textile fabric that is inflexible in the transverse and longitudinal directions.

9. Set according to one of claims 1 to 8, **characterised in that** the application aid is slipper-shaped.

10. Set according to one of claims 1 to 9, **characterised in that** it includes a second compression stocking (10).

## Revendications

1. Ensemble composé d'un bas de compression avec une pointe (12) fermée qui présente sur son côté inférieur une ouverture (13), dans lequel l'ouverture (13) vient se poser après l'application du bas de compression (10) dans la zone des articulations principales des orteils du patient, et une aide à l'enfilage pour un bas de compression, qui est fabriquée en une substance avec une surface lisse et peut être glissée avant l'application du bas de compression (10) sur le pied du patient et peut être retirée par l'ouverture (13) dans la zone de pointe du bas de compression (10) après l'application du bas de compression (10).

2. Ensemble selon la revendication 1, **caractérisé en ce que** l'ouverture (13) présente une forme ronde.

3. Ensemble selon la revendication 1 ou 2, **caractérisé en ce que** l'ouverture (13) présente une largeur dans l'état appliqué du bas de compression (10) qui permet une pénétration de deux bouts de doigt.

4. Ensemble selon l'une quelconque des revendications 1 à 3, **caractérisé en ce que** le bord d'ouverture (14) est renforcé avec une structure de surface textile élastique.

5. Ensemble selon la revendication 4, **caractérisé en ce que** la structure de surface se compose d'un matériau textile élastique, revêtu de substances collantes qui peut être appliqué sous pression et sous l'action de la chaleur.

6. Ensemble selon la revendication 5, **caractérisé en ce que** la structure de surface peut être appliquée au moyen d'une plaque de chauffage, à l'aide d'ultrason ou d'ondes électromagnétiques à haute fréquence.

7. Ensemble selon l'une quelconque des revendications 4 à 6, **caractérisé en ce que** l'ouverture (13) peut être établie après l'application de la structure de surface.

8. Ensemble selon l'une quelconque des revendications 1 à 7, **caractérisé en ce que** l'aide à l'enfilage est fabriquée en une substance textile lisse, non élastique dans le sens transversal et longitudinal.

9. Ensemble selon l'une quelconque des revendications 1 à 8, **caractérisé en ce que** l'aide à l'enfilage est formée comme une pantoufle.

10. Ensemble selon l'une quelconque des revendications 1 à 9, **caractérisé en ce qu'**il contient un second bas de compression (10).
